# NEUE EUROPÄISCHE PATENTSCHRIFT

(11) **EP 0 478 583 B2**
(45) Veröffentlichungstag und Bekanntmachung des Hinweises auf die Entscheidung über den Einspruch: **09.07.1997**
(45) Hinweis auf die Patenterteilung: 04.08.1993
(21) Anmeldenummer: 90908480.8
(22) Anmeldetag: 13.06.1990
(51) Int. Cl.: C02F 1/32, C02F 1/78, A61L 2/10

(54) **VERFAHREN UND ANLAGE ZUR BEHANDLUNG VON MIT SCHADSTOFFEN BELASTETEN FLÜSSIGKEITEN**
PROCESS AND DEVICE FOR TREATING POLLUTED FLUIDS
PROCEDE ET INSTALLATION POUR LE TRAITEMENT DE LIQUIDES CHARGES DE MATIERES POLLUANTES

(30) Priorität: 19.06.1989 DE 3919885
(43) Veröffentlichungstag der Anmeldung: 08.04.1992
(73) Patentinhaber: WEDECO UMWELTTECHNOLOGIE WASSER-BODEN-LUFT GMBH, D-32051 Herford (DE)
(72) Erfinder: LEITZKE, Ortwin, D-4044 Kaarst (DE)
(74) Vertreter: Palgen, Peter, Dr. Dipl.-Phys.
(86) Internationale Anmeldenummer: DE9000446
(87) Internationale Veröffentlichungsnummer: WO9015778

(56) Entgegenhaltungen:
- EP-A- 0 003 879
- EP-A- 0 281 940
- DE-A- 2 551 622
- DE-A- 2 618 338
- US-A- 4 352 740
- US-A- 4 548 716
- US-A- 4 913 827
- B A Beaudet et al.: 'Use of advanced oxidation processes for control of color in groundwater', Veröffentlichung im Rahmen des 'Wasser Berlin '89' - Kongresses des internationalen Ozone Verbandes: IDA, 10-14 April 1989, Berlin, Seiten V-3-1 - V-3-11
- Patent Abstracts of Japan, Band 13, Nr 388, 28 August 1989, & JP-A-01135587
- Journal of Chemical Engeneering of Japan, Vol. 20, Nr.1, Seiten 77-81 (1987)

## Beschreibung

Die Erfindung bezieht sich auf ein Verfahren der dem Oberbegriff des Anspruchs 1 entsprechenden Art sowie eine entsprechende Anlage.

Die Erfindung ist in erster Linie aufein Verfahren zur Behandlung von Flüssigkeiten gerichtet, die mit Schadstoffen belastet sind, die sich schwer oxidieren lassen. Zu dieser Stoffgruppe gehören z.B. die Chlorkohlenwasserstoffe, von denen viele nicht biologisch abbaubarsind und von denen einige sogar toxisch auf Organismen wirken.

Diese Substanzen können gesättigte oder ungesättigte, aliphatische oder aromatische Kohlenwasserstoffe sein, bei denen einige Wasserstoffatome durch Halogene substituiert sind.

Es können niedermolekulare Substanzen, z.B. das Lösungsmittel Trichlorethylen, oder hochmolekulare Lignine oder Huminsäuren sein.

Unter ihnen sind Verbindungen, die mit Ozon, einem der stärksten Oxidationsmittel, langsam odergar überhaupt nicht reagieren.

Diese kaum aufzubrechenden Verbindungen, die in dieser Form nicht dem natürlichen Ökologiekreislauf der Elemente und Stoffe aufunserer Erde zurückgeführt werden können, werden künstlich, chemisch synthetisiert, um z.B. als Treibmittel, Kühlmittel, Lösungsmittel, Pestizide und Herbizide gebraucht zu werden, oder sie entstehen als Nebenprodukte bei irgendwelchen industriellen Prozessen, z.B. bei Chlorbleichprozessen die Chlorlignine.

Sie sickern aus Abfallhalden, vergiften die Grundwässer und die Flüsse. Es besteht die Notwendigkeit, nach Wegen zu suchen, diese Stoffe zu entgiften.

Es ist bekannt, daß UV-Licht von einigen chemischen Verbindungen zwischen den Atomen in bestimmten organischen Molekülen absorbiert wird und daher diese Verbindungen lockert, so daß diese durch Radikale oxidiert, d.h. aufgebrochen werden können.

Solche Oxidantien für energetisch angeregte Verbindungen können OH-Radikale sein. OH-Radikale können durch UV-Bestrahlung wäßriger Lösungen von Wasserstoffsuperoxid (H₂O₂) oder von Ozon (O₃) gebildet werden, indem die Ausgangsverbindungen H₂O₂ und O₃ auch UV-Licht absorbieren und Sauerstoffatome abspalten, die mit dem Wasserzu OH-Radikalen reagieren.

Radikalreaktionen mit H₂O₂ und UV auf organische Verbindungen sind in der Wasseraufbereitung bekannt und auch beschrieben worden (B. Gabel, B. Stachel und W. Thiemann, Fachliche Berichte HWW2, S. 37 - 42, 1982, "Möglichkeiten der technischen Anwendung einer Kombination von Ultraviolett-Bestrahlung und H₂O₂-Behandlung zur Desinfektion von Trinkwasser und Oxidation von Inhaltsstoffen").

Ebenso werden auch Verfahrenskombinationen UV-Bestrahlung und Ozon beschrieben (D. B. Fletcher WaterWorld News, Vol. 3, No. 3, 1987, "UV-/ozone process treat toxics" und K. Brooks, R. McGinty, chemicalweek, Mc. Graw-Hill Publication, "Groundwater treatment know-how comes of age" und J. D. Zeff, E. Leitis, J. Barich, Ca., USA, Ozone in Water Treatment, Vol. 1, Proceedings, 9th Ozone World Congress, New York, 1989, "UV-Oxidation Case Studies on the Removal of Toxic Organic Compounds in Ground, Waste and Leachate Waters", S. 720-731).

Das in diesen Schriftstellen behandelte Ultrox-Verfahren (Ultrox = eingetragenes Warenzeichen der Fa. Ultrox International, Santa Ana, Ca, V.St.v.A.) hat ein UV-Oxidantien-Kontaktsystem für einen kontinuierlichen oder einen diskontinuierlichen Flüssigkeitsstrom. Die UV-Strahler sind vertikal in unterschiedlichster Zahl in mehreren Kammern, die aneinandergereiht sind, montiert. In diesen Kammern fließt oder steht das Wasser und umgibt die mit QuarzSchutzrohren geschützten Lampen. Ozon oder andere Oxidantien werden durch Stahidiffusoren in die Kammern eingetragen.

Das APO-Verfahren (APO = eingetragenes Warenzeichen der Fa. Ionization International, Dordrecht, Niederlande) (J.-A. Moser, M. Sc., Ozone in Water Treatment, Vol. 1, Proceedings, 9th Ozone World Congress, New York, 1989, S. 732 - 742, "The Treatment of Chlorinated Hydrocarbons at a High Concentration Level with a Photochemical Process") produziert Ozon mit UV-Licht kurzer Wellenlänge und läßt das Ozon in der Wasser- oder in der Gasphase auf Chlorkohlenwasserstoffe einwirken.

Das Problem bei den H₂O₂-UV-Kombinationsver-fahren liegt darin, daß sie nicht so hohe Oxidationspotentiale erreichen, wie es bei den O₃-UV-Kombinationen möglich ist.

Die bisher bekannten O₃-UV-Kombinationsverfahren arbeiten mit UV-Tauchstrahlern, wie z.B. Ultrox. Diese haben in den Bestrahlungskammern große Wasserschichtdicken, die schwerer mit UV-Strahlen zu durchdringen sind als bei Durchlaufstrahlern mit dünneren Wasserschichten. Außerdem wird bei diesen Kammern das Ozon mit dem Trägergas direkt eingetragen, wobei die Ozonlösung im Wasser nicht optimal ist, und die Gasblasen des nicht physikalisch gelosten Gases sich ebenfalls nachteilig für eine UV-Strahlen-Ausnutzung auswirken.

Die Verfahren, die Ozon durch UV-Strahlen, wie das APO-Verfahren, oder durch elektrolytische anodische Oxidation erzeugen, produzieren Ozon nur in geringer Konzentration, was nachteilig für das Oxidationsvermögen ist.

Ein Verfahren dieser Art ist Gegenstand der EP 281 940 A1. Gegenstand dieser Schrift ist eine "Wasseraufbereitung für Sonderzwecke, wo extreme Bedingungen bezüglich Reinheit in chemischer, physikalischer und biologischer Hinsicht zu erfüllen sind". Das behandelte Wasser soll zur Weiterverwendung für bestimmte Zwecke präpariert werden, nämlich für Spülzwecke in der Fabrikation von Halbleiterbauteilen der Elektronikindustrie. Die für diese Zwecke bei der EP 281 940 A1 benötigten Reinstwassermengen sind wesentlich geringer als die bei der Behandlung belasteter Abwässer oder Flüssigkeiten durchzusetzenden Mengen. Bei der EP 281 940 A1 wird das Ozon auf elektrischem Wege durch Zerlegung der zu behandelnden Flüssigkeit erzeugt. Dieser Weg zur Bereitstellung des Ozons ist ein integrierendes Merkmal der EP 281 940 A1, weil das bei der Elektrolyse entstehende H₂ im weiteren Verfahren wieder gebraucht wird, um das in dem Wasser verbliebene O₃ wieder abzubauen bzw. um jeglichen Sauerstoffgehalt in dem erzeugten Reinstwasser zu vermeiden.

Etwa nicht gelöstes O₃ kann aufgefangen und in O₂ zurückverwandelt werden, um es unschädlich zu machen.

Das Wasser wird nach der Ozonisierung einer UV-Bestrahlung unterworfen.

Für die Behandlung von belasteten Abwässern kommt es nicht auf die Erzielung von Reinstwasser, sondern nur darauf an, daß das Wasser anschließend in den allgemeinen Wasserkreislauf entlassen werden kann. Andererseits sind die Mengen so groß, daß die auf elektrochemischem Wege bereitstellbaren Ozonmengen nicht entfernt ausreichen.

Aus der Schriftstelle Kiyoshi Ikemizu, Shigeharu Morooka und Yasuo Kato im Journal of Chemical Engineering of Japan, Vol. 20, Nr. 1, 1987, Seiten 77-81, die den oberbegriffen der Ansprüche 1 und 11 zugrundeliegt, wird über einen Laborversuch berichtet, bei welchem eine Mischung aus Ozon und Sauerstoff in eine Blasensäule von 4 cm Durchmesser und 25 cm Höhe eingeblasen wurde. Die ozongesättigte Lösung wurde aus dem Gasblasenraum heraus kontinuierlich in einen UV-Reaktor gepumpt, der einen zwischen zwei Quarzglasplatten ausgebildeten Reaktionsraum umfaßte, der von der Lösung durchströmt und quer dazu von der Strahlung einer UV-Lampe durchstrahlt wurde, die hauptsächlich Licht der Wellenlänge 253,7 nm abgab.

Es handelt sich um eine Versuchsanordnung, die der Umsetzung in den technischen Maßstab bedarf. Die Art der Entnahme der Lösung aus der Glasblasensäule läßt erkennen, daß die Lösung mit samt einem erheblichen Anteil an ungelöstem, in Form kleiner Gasbläschen vorhandenen ozonhaltigen Gases durch den UV-Reaktor geleitet wurde, was dessen Wirksamkeit stark herabsetzt.

Der Erfindung liegt die Aufgabe zugrunde, die Wirksamkeit der kombinierten UV-Ozon-Behandlung zu verbessern.

Diese Aufgabe wird in ihrem verfahrensmäßigen Aspekt durch die in Anspruch 1 wiedergegebene Erfindung gelöst.

Die Ozonerzeuger mit stiller Entladung werden aus Gründen der hohen Ausbeute verwendet.

Durch die Abtrennung des nicht gelösten Gases ist erreicht, daß die Flüssigkeit in praktisch blasenfreiem Zustand mit physikalisch gelöstem Ozon der UV-Bestrahlung zugeführt wird, wodurch deren Wirkung sich wesentlich intensiviert.

Gemäß der Erfindung empfiehlt es sich, das ozonhaltige Gas unter erhöhtem Druck in die Flüssigkeit einzuleiten und auch den Systemdruck insgesamt zu erhöhen, wodurch der Ozonpartialdruck in der Flüssigkeit und auch die Löslichkeit des Ozons gesteigert werden, was der Wirksamkeit der Ozonbehandlung zugute kommt.

Eine wichtige Ausgestaltung der Erfindung besteht in der Mehrfachbehandlung der Flüssigkeit in einem Kreislauf, wobei der im Kreislauf geführte Flüssigkeitsstrom größer ist als der kontinuierlich zu- und ablaufende Flüssigkeitsstrom.

Hierdurch wird eine möglichstvollständige Ozonaufnahme und eine bessere UV-Transmission durch den Verdünnungseffekt erreicht. Außerdem erfolgteine mehrfache Beaufschlagung mit dem UV-Licht in ein und derselben Anlage und eine Verlängerung der Verweilzeit im Bestrahlungsbereich.

Gemäß Anspruch 2 kann die Einleitung des ozonhaltigen Gases in den Zulauf der zu behandelnden Flüssigkeit oder einen Teil des Zulaufs erfolgen.

Um die Ausbeute an in Lösung gebrachtem Ozon zu erhöhen, empfiehlt es sich, daß das abgetrennte restozonhaltige Ozonträgergas erneut mit der Flüssigkeit reagieren gelassen wird (Anspruch 3).

Wenn technischer Sauerstoff zur Ozonerzeugung benutzt wird und das Ozonträgergas also Sauerstoff ist, kann gemäß Anspruch 4 dieser Sauerstoff nach der Abtrennung von dem Ozon über eine Trocknungsanlage der Ozonquelle bzw. der Ozonerzeugung wieder zugeführt werden.

Eine zweckmäßige Ausgestaltung besteht gemäß Anspruch 5 in einer Bestrahlung mit UV-Licht unterschiedlicher Wellenlänge, die gleichzeitig (Anspruch 6) oder nacheinander auf ein bestimmtes Flüssigkeitsvolumen einwirken können (Anspruch 7). Die Wellenlängen können dabei diskret sein, z.B. die meistverwendete Wellenlänge 254 nm und andere bestimmte Werte, oder aber auchein kontinuierliches Wellenlängenband umfassen, allein oder in beliebiger Kombination.

Durch den Unterschied in den Wellenlängen kann eine Anpassung des Energieeintrags an die verschiedenen mit den Schadstoffen stattfindenden Reaktionen erfolgen.

Gemäß Anspruch 8 kann der pH-Wert der zu behandelnden Flüssigkeit reguliert werden, um die Reaktivität derselben zu steigern.

Entsprechend ist es möglich, die zu behandelnde Flüssigkeit zu beheizen, um die Reaktionsgeschwindigkeit zu steigern.

Durch die Ausgestaltung des Verfahrens nach Anspruch 10 ist erreicht, daß allein mit Ozon reaktionsfähige Substanzen, z.B. Azofarben, Kolloide und Trübstoffe vor der kombinierten gleichzeitigen Einwirkung von Ozon und UV zerstört bzw. abgeschieden werden, so daß die Klarheit der Flüssigkeit und damit die Durchlässigkeit für das UV-Licht und dessen Wirksamkeit zunehmen.

In dem vorrichtungsmäßigen Aspekt wird die Erfindung durch eine Anlage nach Anspruch 11 verwirklicht.

Der Reaktions- und Entgasungsbehälter, in welchem einerseits die Reaktion des Ozons mit den Schadstoffen und andererseits die Austreibung und Abtrennung der nicht gelösten Anteile des ozonhaltigen Gases stattfindet, ist in der bevorzugten Ausführungsform gemäß Anspruch 12 ausgebildet.

Die Flüssigkeit mit dem eingeleiteten ozonhaltigen Gas wird so geführt, daß das Gemisch zunächst in den inneren Behälter gelangt, aus welchem das nicht physikalisch gelöste Gas entweichen kann, welches an der Abzugsleitung entfernt wird. Die Flüssigkeit steigt durch die Zuführung im unteren Bereich des inneren Behälters in diesem aufund strömt in den äußeren Behälter über und wird aus der beruhigten Zone im unteren Bereich des äußeren Behälters abgeleitet.

Es können gemäß Anspruch 13 Ableitungen sowohl für die Ablauf- als auch für die Kreislaufflüssigkeit vorgesehen sein, die übereine Rückführleitung in den Zulauf zurückgeführt wird.

Dieser Kreislauf, der eine mehrfache Behandlung ein und desselben Flüssigkeitsvolumens ermöglicht, istein wesentliches Ausgestaltungsmerkmal der Erfindung.

Ein weiteres wesentliches Ausgestaltungsmerkmal ist das Vorhandensein mehrerer hintereinandergeschalteter Reaktions- und Entgasungsbehälter zur Intensivierung der Einwirkung (Anspruch 14).

In einer bevorzugten Ausführungsform umfaßt die Einrichtung zur Bestrahlung der zu behandelnden Flüssigkeit eine zwischen die beiden Reaktions- und Entgasungsbehälter eingeschaltete Bestrahlungseinheit (Anspruch 15).

Es kann aber auch eine Bestrahlungseinheit in der Ableitung des letzten Reaktions- und Entgasungsbehälters und/oder in der von den Ableitungen der Reaktions- und Entgasungsbehälter in den Zulauf zurückführenden Rückführleitung angeordnet sein (Anspruch 16,17).

Um die Ausbeute an in Lösung gebrachtem Ozon zu verbessern, kann die Ausgestaltung nach Anspruch 18 vorgesehen sein.

Das in dem ersten Reaktions- und Entgasungsbehälter abgetrennte Gas kann noch Ozonanteile enthalten, die durch die angegebene Maßnahme in dem zweiten Reaktions- und Entgasungsbehälter noch zur Wirkung gebracht werden können.

Das Ozon kann mittels einer im Zulauf der Flüssigkeit angeordneten Einleitungsvorrichtung (Anspruch 19) und/oder in einer in der Rückführleitung angeordneten Einleitungsvorrichtung (Anspruch 20) in die Flüssigkeit eingeleitet werden.

Zur Intensivierung der UV-Einwirkung empfiehlt es sich, UV-Bestrahlungseinheiten solcher konstruktiver Ausgestaltung zu verwenden, daß eine strömende Flüssigkeitsschicht relativ geringer Dicke quer durchstrahlt wird.

Als Ozonerzeuger werden aus Gründen der hohen Ausbeute zweckmäßig solche verwendet, die mit stiller Entladung arbeiten (Anspruch 25).

In der Zeichnung sind Ausführungsbeispiel der Erfindung dargestellt.

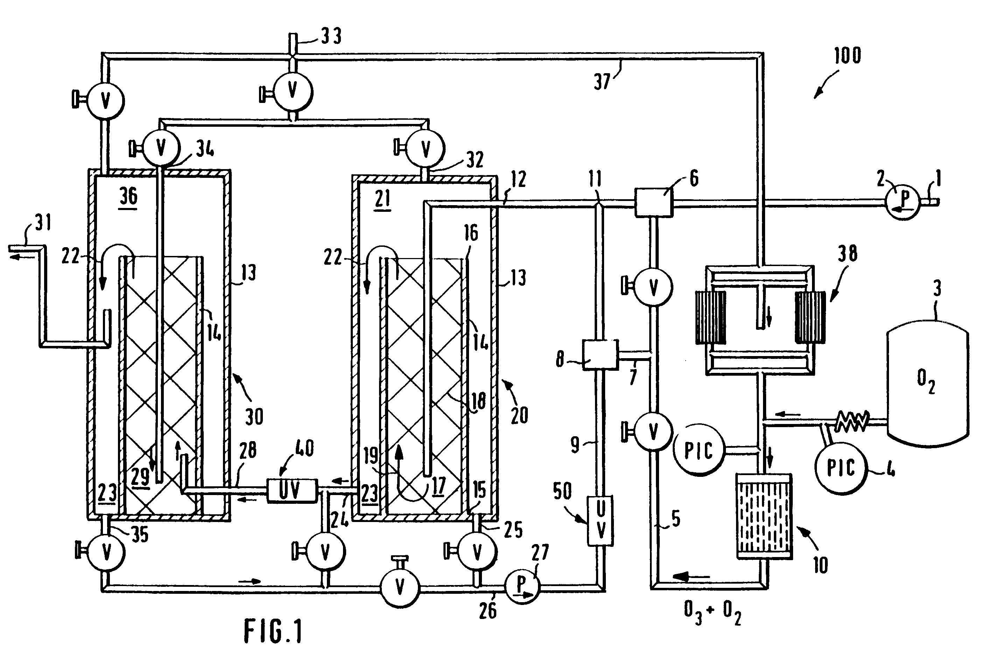

Fig. 1 bis 3 zeigen schematische Diagramme dreier Anlagen zur Behandlung von mit Schadstoffen belastetem Wasser.

Die in Fig. 1 dargestellte als Ganzes mit 100 bezeichnete Anlage umfaßt im wesentlichen einen Ozonerzeuger 10, einen ersten Reaktions- und Entgasungsbehälter 20, einen zweiten Reaktions- und Entgasungsbehälter 30, eine UV-Bestrahlungseinheit 40 und eine zweite UV-Bestrahlungseinheit 50.

Das die zu behandelnde Flüssigkeit bildende Rohwasser tritt am Zulauf 1 in die Anlage ein und wird durch eine Pumpe 2 auf einen erhöhten Druck von einigen Bar gebracht.

In dem Ausführungsbeispiel wird das Ozon aus Sauerstoffgewonnen. Das Sauerstoffgas wird einem Drucktank 3 entnommen und über einen Druckminderer 4 durch den Ozonerzeuger 10 geschickt, in welchem Ozon in einer Konzentration von ca. 100 g/m³ Sauerstoff erzeugt wird. Das aus O₃ und O₂ bestehende sauerstoffhaltige Gas wird über die Leitung 5 dem Injektor 6 zugeführt, der in dem Zulauf 1 der Flüssigkeit angeordnet ist In dem Injektor 6 wird also das ozonhaltige Gas, welches seinerseits unter leicht erhöhtem Druck steht, in die unter erhöhtem Druck stehende Flüssigkeit eingesaugt

Über eine Zweigleitung 7 gelangt ozonhaltiges Gas auch zu dem Injektor 8, der in einer Rückleitung 9 angeordnet ist, die an einer Stelle 11 stromabwärts des Injektors 6 in den Zulauf 1 mündet.

Die durch die Injektoren 6 und 8 mit ozonhaltigem Gas versetzte Flüssigkeit gelangt über die Zuleitung 12 in den Reaktion- und Entgasungsbehälter 20.

Der Reaktions- und Entgasungsbehälter 20 besteht in dem Ausführungsbeispiel aus einem zylindrischen äußeren Behälter 13, in welchem konzentrisch ein zylindrischer innerer Behälter 14 angeordnet ist. Der innere Behälter 14 ist bei 15 am unteren Rand dicht mit dem Boden des äusseren Behälters 13 verbunden. Am oberen Rand 16 ist der innere Behälter 14 offen und bildet einen Überlauf. Die Zuleitung 12 führt in den unteren Bereich 17 des inneren Behälters 14, der mit Füllkörpern und/oder Schikanen versehen ist, die durch die Kreuzschraffur 18 angedeutet sind. Die Flüssigkeit strömt also im Sinne des Pfeiles 19 durch den Behälter 14 nach oben und wird vielfach turbulent umgelenkt, so daß das in die Flüssigkeit eingeleitete Ozon Gelegenheit hat zu reagieren und in die Flüssigkeit nur ein gemischtes, aber nicht physikalisch gelöstes Gas Gelegenheit hat sich zu entlösen und in den Raum 21 oberhalb des inneren Behälters aufzusteigen. Die Flüssigkeit strömt sodann über den oberen Rand 16 im Sinne des Pfeiles 22 in dem äußeren Behälter nach unten. In dem unteren Bereich 23 des äußeren Behälters befindet sich eine Beruhigungszone, in der die Ozonreaktion und die Entgasungsreaktion schon zu einem wesentlichen Teil abgelaufen sind. In dem unteren Bereich 23 finden sich an dem äußeren Behälter 13 Ableitungen 24, durch die Flüssigkeit in die UV-Bestrahlungseinheit 40 übertritt, und 25, durch die Flüssigkeit in die Leitung 26 übertritt, in der eine Pumpe 27 angeordnet ist, die die Flüssigkeit wieder auf Druck bringt. Von der Pumpe 27 strömt die Flüssigkeit durch die UV-Bestrahlungseinheit 50 und überdie Rückleitung 9 zu dem Injektor 8 zurück.

Die Flüssigkeitsanteile, die die UV-Bestrahlungseinheit 40 passiert haben, gelangen über die Zuleitung 28 in den unteren bereich 29 des zweiten Reaktions- und Entgasungsbehälters 30, der in dem Ausführungsbeispiel genauso ausgebildet ist wie der Reaktions- und Entgasungsbehälter 20. Die Flüssigkeit steigt in dem inneren Behälter des Reaktions- und Entgasungsbehälters 30 empor, wobei die Restreaktion mit dem gelösten Ozon stattfindet. Nach dem Überströmen des oberen Randes des inneren Behälters 14 gelangt die fertigbehandelte Flüssigkeit in den Ablauf 31. Ein anderer Anteil der im Sinne des Pfeiles 22 übergeströmten Flüssigkeit wird jedoch durch die Ableitung 35 abgezogen und gelangt in die Leitung 26 und durchsetzt erneut den Injektor 8.

Das bei der Entgasung in dem Reaktions- und Entgasungsbehälter 20 abgetrennte restozonhaltige Ozonträgergas sammelt sich im oberen Bereich 21 des geschlossenen äußeren Behälters 13 an und wird von dort über die Abzugsleitung 32 abgezogen. Dieses Gas kann entweder über die Leitung 33 abgeblasen oder aber über eine Zuleitung 34 in den unteren Bereich 29 des zweiten Reaktions- und Entgasungsbehälters 30 eingeleitet werden, von wo es in der Flüssigkeit in dem inneren Behälter 14 aufsteigt, so daß die Restozonanteile eine erneute Gelegenheit zur Reaktion haben.

Die in dem oberen Bereich 36 sich ansammelnden Mengen des Ozonträgergases, welches in dem Ausführungsbeispiel aus Sauerstoff besteht, können über die Leitung 37 und einen Gastrockner 38 in den Ozonerzeuger 10 zurückgeführt werden. Anstelle von Sauerstoff können auch andere Gase wie Luft, Stickstoff oder Argon als Ozonträgergase dienen.

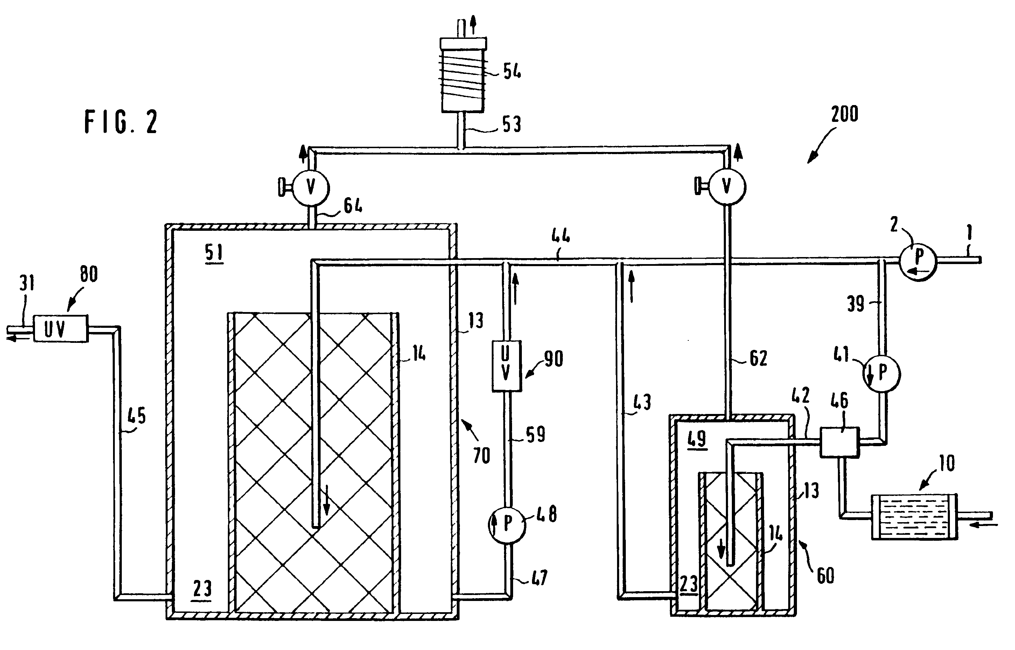

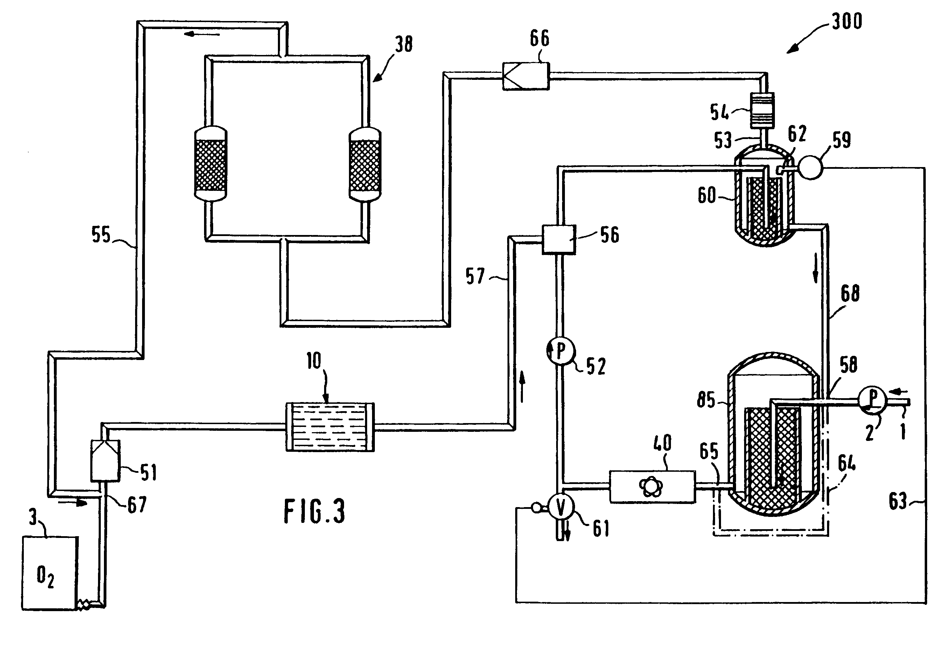

Soweit bei den Ausführungsformen 200 und 300 nach den Fig. 2 bzw. 3 funktionell enstsprechende Komponenten vorhanden sind, sind die Bezugszahlen gleich.

Die bei der Ausführungsform 200 der Fig. 2 am Zulauf 1 eingetretene und durch die Pumpe 2 auf Systemdruck gebrachte Rohwassermenge wird stromaufwärts der Pumpe 2 aufgeteilt. Der abgeteilte Zweigstrom in der Leitung 39 wird durch eine Druckerhöhungspumpe 41 auf höheren Druck gebracht und durch eine Leitung 42 einem Reaktions- und Entgasungsbehälter 60 zugeführt, dessen Aufbau und Wirkungsweise im wesentlichen dem Reaktions- und Entgasungsbehälter 20 entspricht. Flüssigkeit aus dem unteren Bereich des äußeren Behälters 13 des Reaktions- und Entgasungsbehälters 60 wird über die Leitung 43 wieder dem Hauptstrom in der Leitung 44 zugemischt, die die Zuleitung zu einem zweiten Reaktions- und Entgasungsbehälter 70 bildet, der ebenfalls entsprechend dem Reaktions- und Entgasungsbehälter 20 ausgebildet ist und funktioniert. Aus dem unteren Bereich 23 des äußeren Behälters 13 gelangt Flüssigkeit nach Reaktion mit dem Ozon in den Ablauf 31, wobei in der Zuleitung 45 zu dem Ablauf 31 eine UV-Bestrahlungseinheit 80 angeordnet ist, die die Flüssigkeit einer abschließenden UV-Bestrahlung aussetzt.

Aus dem unteren Bereich 23 führt eine weitere Leitung 47 über eine Pumpe 48 und eine Leitung 59 zu einer weiteren UV-Bestrahlungseinheit 90, aus der die Flüssigkeit in den Hauptstrom in der Leitung 44 zurückgelangt. Über die Leitungen 47,44 wird Flüssigkeit mit gelöstem Ozon mehrfach im Kreislauf umgepumpt und dabei wiederholt der UV-Bestrahlung in der Bestrahlungseinheit 90 ausgesetzt.

Das sich überder Flüssigkeit in den Bereichen 49 und 51 ansammelnde Ozonträgergas wird über die Leitungen 62,64 und 53 ins Freie abgelassen, nachdem es einen Restozonumwandler 54 passiert hat.

Bei der Ausführungsform 300 nach Fig. 3 tritt die zu behandelnde Flüssigkeit in Gestalt eines Abwassers über die Leitung 1 in die Anlage ein und wird durch die Pumpe 2 auf Druck gebracht. Sie durchströmt den Reaktionsbehälter 85 und danach die UV-Bestrahlungseinheit 40. Anschließend wird sie von einer weiteren Pumpe 52 wieder auf Druck gebracht und passiert einen Injektor 56, in welchem über eine Leitung 57 herangeführtes ozonhaltiges Gas in die Flüssigkeit eingemischt wird. Die mit dem Ozon versetzte Flüssigkeit gelangt dann in den Entgasungsbehälter 60, wo der nicht in der Flüssigkeit gelöste restozonhaltige Gasanteil abgetrennt wird, der über eine Leitung 53 abgezogen wird. Die gelöstes Ozon enthaltende Flüssigkeit wird über die Leitung 56 dem Punkt 58 zugeführt, wo sie dem eingeleiteten Rohwasser beigemischtwird. Die Ozonreaktion beginnt in dem Reaktionsbehälter 85, der vorhanden ist, wenn ein hoher Anteil an ionischer Reaktion für die Behandlung des individuellen Rohwassers erforderlich ist.

Es ist in bestimmten Fällen jedoch auch möglich, den Reaktionsbehälter 85 wegzulassen und von dem Punkt 58 über die strichpunktiert wiedergegebene Leitung 64 an einen Punkt 65 hinter dem Reaktionsbehälter 85 und von dort unmittelbar in die UV-Bestrahlungseinheit 40 zu gehen. Nach der Beimengung des Ozons in dem Injektor 56 erfolgt die Entgasung in dem Entgasungsbehälter 60 und wird die entgaste, Ozon nur in gelöster Form enthaltende Flüssigkeit über die Leitungen 56 und 64 wieder der UV-Bestrahlungseinheit 40 zugeleitet. Die mit Ozon versehenen Flüssigkeitsanteile passieren also die UV-Bestrahlungseinheit 40 wiederum nur in entgaster Form, was die Wirksamkeit der Bestrahlung erhöht.

Das Ozon wird aus Sauerstoff gebildet, der in einem Drucktank 3 bevorratet ist und über einen Filter in den Ozonerzeuger 10 gelangt. Das Produkt des Ozonerzeugers 10 ist eine Mischung aus verbliebenem O₂ als Trägergas und einigen Prozent O₃. Diese Mischung wird überdie Leitung 57 dem Injektor 56 zugeführt.

Das dem Entgasungsbehälter 60 über die Leitung 53 entnommene nicht gelöste restozonhaltige Gas wird in dem Restozonumwandler 54 behandelt, worin das verbliebene Ozon in O₂ zurückverwandelt wird, welches über einen Filter 66 und einen als Ganzes mit 38 bezeichneten Gastrockner über die Leitung 55 an die Stelle 67 vor dem Filter 51 zurückgeführt und erneut einer Ozonisation in dem Ozonerzeuger 10 ausgesetzt wird.

Die Menge der in der Anlage befindlichen Flüssigkeit kann mittels eines Niveaureglers 59 konstant gehalten werden, der mit einem Niveaufühler 62 in dem Entgasungsbehälter 60 zusammenwirkt und über eine Steuerleitung 63 ein Ventil 61 ansteuert, welches beim Steigen des Niveaus in dem Entgasungsbehälter 60 Flüssigkeit abläßt.

Die Bestrahlungseinheiten 40,50,80,90 sind von einer solchen Bauart, daß sie von der Flüssigkeit in einer relativ dünnen Schicht durchströmt werden und die Schwächung des die Flüssigkeitsschicht quer durchsetzenden UV-Lichts gering ist.

Die Anlagen erzielen ihre besondere kombinierte Wirkung von Ozon und UV-Bestrahlung dadurch, daß
- Ozongas in hohen Konzentrationen unter Druck mit Hilfe von Wasserdruckerhöhungspumpen und Injektoren in die Flüssigkeit eingetragen wird,
- Die Löslichkeit des Gases nicht nur durch die Erhöhung des Ozonpartialdruckes (hohe Ozonkonzentration und hoher Systemwasserdruck), sondern auch durch die Erhöhung des Wasservolumens zum Ozoneintrag gegenüber dem Rohwasserzufluß durch mehrfache Wasserkreislaufführungen und Schikanen im Entgasungs- und Reaktionstank gesteigert wird.
- durch die zuerst genannten beiden Punkte blasenfreies Wasser, in dem Ozon bereits physikalisch gelöst ist, der UV-Bestrahlung zugeführt wird,
- durch die Vervielfachung des Kreislaufwassers, was bestrahlt wird, gegenüber dem konzentrierten kleineren Rohwasserstrom eine Verdünnung und Verbesserung der UV-Transmission erreicht wird,
- durch die Durchfluß-UV-Bestahlungsapparate mit positiver Bestrahlungsgeometrie und dünnen Flüssigkeitswasserschichten die Wirkung des UV-Lichtes auf die Flüssigkeitsinhaltsstoffe und das gelöste Ozon gut ist,
- durch die Flüssigkeitskreislaufführung eine mehrfache Dosierung des UV-Lichtes mit einem Apparat und einer Verweilzeitverlängerung im Bestrahlungsbereich möglich sind und
- durch den Ozoneintrag vor dem Entgasungsbehälter und der beginnenden Reaktion des Ozons auf dem Wege zur Einsatzstelle der kombinierten gleichzeitigen Wirkung von Ozon und UV mit den allein durch ozonreaktionsfähigen Substanzen, z.B. Farben und Trübstoffe, die Klarheit der Flüssigkeit und damit der Transmission des UV-Lichtes und der kombinierten Wirkung von Ozon und UV zunehmen.

Generell kann durch eine Ozon-UV-Kombination eine Oxidation schwer abbaubarer Substanzen, die teilweise sogar toxisch sind, soweit erfolgen, daß entweder die geforderten Grenzwerte erreicht oder sogar die Substanzen weiter biologisch abgebaut werden können, oder sogar eine Mineralisierung erfolgt, wenn genügend hohe Dosen angewandt werden.

Verfahrensbedingte Vorteile dieser Erfindung gegenüber ähnlichen Verfahren sind aber:
- kein Ausstrippen von toxischen Substanzen durch mehrfache Zwangsgasführung,
- guter Ozonlöslichkeitsfaktor durch mehrere Lösungsstufen,
- Angebot gasblasenfreier ozonbeladener Flüssigkeit für UV-Bestrahlung,
- hoher UV-Dosis-Eintrag durch Mehrfachkreislaufführung der Flüssigkeit mit einem Bestrahlungsapparat,
- UV-Transmissionserhöhung der Zulaufflüssigkeit durch Verdünnen und durch eventuelle Reaktion des Ozons mit Farbstoffen,
- installierte Kombinationsmöglichkeit:
   zur pH-Wertanhebung
   zur Temperaturerhöhung und
   Flockung durch Ozon
- und Variationsmöglichkeit des UV-Bestrahlungsspektrums durch unterschiedliche Strahlungsquellen, womit das Strahlungsoptimum an das Absorptionsoptimum organischer Substanzen angeglichen werden kann.

Durch die Kombination dieses Verfahrens mit einer biologischen Stufe können, wie bereits in der Erprobung bewiesen, die im Augenblick in der Diskussion stehenden Grenzwerte für eine Verwaltungsvorschrift der Sickerwasserabläufe von 500 µg pro Liter AOX pro cbm Ablaufwasser um eine Zehnerpotenz unterschritten werden, so daß neue Standards der Technologie gesetzt werden.

## Patentansprüche

1. Verfahren zur Behandlung von mit schwer abbaubaren Schadstoffen belasteten wässrigen Flüssigkeiten durch nasse Oxidation mit ozonhaltigem Gas und anschließende Ozon-UV-Behandlung,
**dadurch gekennzeichnet,**
daß das ozonhaltige Gas aus sauerstoffhaltigem Gas in einem Ozonerzeuger durch stille Entladung erzeugt wird,
daß das ozonhaltige Gas in einem Teil der Anlage, der nicht der UV-Bestrahlung ausgesetzt ist, unter erhöhtem Druck von einigen Bar in die wässrige Flüssigkeit eingeleitet wird,
daß das nicht gelöste ozonhaltige Gas vor der UV-Bestrahlung abgetrennt wird,
daß die wässrige Flüssigkeit bei der Einleitung des ozonhaltigen Gases und bei der Abtrennung des nicht gelösten ozonhaltigen Gases unter dem erhöhten Druck gehalten wird,
daß die im wesentlichen gasblasenfreie, Ozon in absorbierter Form enthaltende wässrige Flüssigkeit mit UV-Licht zum Zwecke der gleichzeitigen Radikalbildung und Oxidation mit Radikalen im Durchfluß durch einen UV-Licht erzeugenden Apparat durchstrahlt wird,
und daß die wässrige Flüssigkeit in einem Kreislauf mehrfach behandelt wird, wobei der im Kreislauf geführte Flüssigkeitsstrom größer als der kontinuierlich zu- und ablaufende Flüssigkeitsstrom ist.

2. Verfahren nach Anspruch 1, **dadurch gekennzeichnet**, daß die Einleitung des ozonhaltigen Gases mit Hilfe eines Injektors in den Zulauf der zu behandelnden Flüssigkeit oder einen Teil des Zulaufs erfolgt.

3. Verfahren nach Anspruch 1 oder 2, **dadurch gekennzeichnet**, daß das abgetrennte restozonhaltige Ozonträgergas zur Erzielung einer Restozonreaktion erneut mit der Flüssigkeit reagieren gelassen wird.

4. Verfahren nach einem der Anspruch 1 bis 3, **dadurch gekennzeichnet**, daß technischer Sauerstoff zur Ozonerzeugung benutzt und das restozonhaltige Sauerstoffgas nach der Abtrennung von der Flüssigkeit getrocknet und zur Ozonerzeugung wiederbenutzt wird.

5. Verfahren nach einem der Ansprüche 1 bis 4, **dadurch gekennzeichnet**, daß die UV-Bestrahlung mit UV-Licht unterschiedlicher Wellenlänge erfolgt.

6. Verfahren nach Anspruch 5, **dadurch gekennzeichnet**, daß die Bestrahlung eines bestimmten Flüssigkeitsvolumens mit den unterschiedlichen Wellenlängen gleichzeitig erfolgt.

7. Verfahren nach Anspruch 5, **dadurch gekennzeichnet**, daß die Bestrahlung eines bestimmten Flüssigkeitsvolumens mit den unterschiedlichen Wellenlängen nacheinander erfolgt.

8. Verfahren nach einem der Ansprüche 1 bis 7, **dadurch gekennzeichnet**, daß der pH-Wert der zu behandelnden Flüssigkeit reguliert wird, um die Reaktivität derselben zu steigern.

9. Verfahren nach einem der Ansprüche 1 bis 8, **dadurch gekennzeichnet**, daß die zu behandelnde Flüssigkeit beheizt wird, um die Reaktionsgeschwindigkeit zu steigern.

10. Verfahren nach einem der Ansprüche 1 bis 9, **dadurch gekennzeichnet**, daß nach dem Einleiten des ozonhaltigen Gases und vor der Ozon-UV-Kombinationseinwirkung die Flüssigkeit einer Flockungsfiltration bzw. einer Flockungssedimentation unterworfen wird.

11. Anlage zur Durchführung des Verfahrens nach den Ansprüchen 1 bis 10,
mit einem Zulauf (1) für die zu behandelnde wässrige Flüssigkeit und einem Ablauf (31) für die fertig behandelte wässrige Flüssigkeit,
mit einer Quelle ozonhaltigen Gases,
mit einer Einrichtung zur Einleitung des ozonhaltigen Gases in die zu behandelnde Flüssigkeit
und mit einer der Einrichtung zur Einleitung des ozonhaltigen Gases nachgeschalteten Einrichtung zur Bestrahlung der zu behandelnden Flüssigkeit mit UV-Licht,
**dadurch gekennzeichnet**,
daß die Quelle ozonhaltigen Gases ein Ozonerzeuger (10) ist, in welchem aus einem sauerstoffhaltigen Gas das ozonhaltige Gas durch stille Entladung erzeugbar ist,
daß eine Einrichtung (6,8;46) zur Einleitung des ozonhaltigen Gases in die zu behandelnde wässrige Flüssigkeit unter erhöhtem Druck von einigen Bar vorgesehen ist,
daß eine der Einrichtung (6,8;46) nachgeschaltete Einrichtung (40,50;80,90) zur Bestrahlung der zu behandelnden Flüssigkeit mit UV-Licht vorgesehen ist,
daß zwischen den Einrichtungen (6,8;46) ein Reaktions- und Entgasungsbehälter (20;60,70) angeordnet ist
und daß zur Führung der wässrigen Flüssigkeit durch die Einrichtungen (6,8;46) und (40,50;80,90) und die Reaktions- und Entgasungsbehälter (20,30;60,70) ein Kreislauf vorgesehen ist, in welchem eine größere Flüssigkeitsmenge führbar ist, als im kontinuierlichen Betrieb am Zulauf (1) zuläuft und am Auslauf (31) abläuft.

12. Anlage nach Anspruch 11, **dadurch gekennzeichnet**, daß die Reaktions- und Entgasungsbehälteranordnung Reaktions- und Entgasungsbehälter (20,30,60,70) umfaßt, die als Doppelbehälter mit zwei ineinanderstehenden Behältern (13,14) ausgebildet sind, von denen der äußere Behälter (13) bis auf eine im oberen Bereich (21,36,49,51) einmündende Abzugsleitung (32,34,62,53) für das abgetrennte Ozonträgergas geschlossen und der innere Behälter (14) oben offen ist und einen Überlauf (16) bildet, wobei die Zuleitung (12,42,44) für die zu behandelnde Flüssigkeit von oben bis in den unteren Bereich (17) des inneren Behälters (14) geführt ist und die Ableitungen (24,25,35,43,45,47) vom unteren Bereich (23,79) des äußeren Behälters (13) ausgehen.

13. Anlage nach Anspruch 11 oder 12, **dadurch gekennzeichnet**, daß in dem unteren Bereich (23) des äußeren Behälters (13) Ableitungen (24,31 bzw. 25,35,47) sowohl für den Ablauf bestimmte Flüssigkeit als auch für für den Kreislauf bestimmte Flüssigkeit, die über eine Rückführleitung (26,47) in den Zulauf zurückgeführt wird, vorgesehen sind.

14. Anlage nach einem der Ansprüche 11 bis 13, **dadurch gekennzeichnet**, daß einem ersten Reaktions- und Entgasungsbehälter (20,60) mindestens ein weiterer Reaktions- und Entgasungsbehälter (30,70) nachgeschaltet ist, dessen Zuleitung (28,44) mit der Ableitung (24,43) des ersten Reaktions- und Entgasungsbehälters (20,60) verbunden ist.

15. Anlage nach Anspruch 14, **dadurch gekennzeichnet**, daß die Einrichtung zur Bestrahlung der zu behandelnden Flüssigkeit eine zwischen die beiden Reaktions- und Entgasungsbehälter (20,30) eingeschaltete Bestrahlungseinheit (40) umfaßt.

16. Anlage nach einem der Ansprüche 11 bis 15, **dadurch gekennzeichnet**, daß die Einrichtung zur Bestrahlung der zu behandelnden Flüssigkeit eine in der Ableitung (45) des letzten Reaktions- und Entgasungsbehälters (70) angeordnete Bestrahlungseinheit (80) umfaßt.

17. Anlage nach einem der Ansprüche 11 bis 16, **dadurch gekennzeichnet**, daß die Einrichtung (90) zur Bestrahlung der zu behandelnden Flüssigkeit eine in eine von den Ableitungen (25,35;47) in den Zulauf zurückführenden Rückführleitung (9,59) angeordnete Bestrahlungseinheit (90) umfaßt.

18. Anlage nach einem der Ansprüche 14 bis 17, **dadurch gekennzeichnet**, daß eine Verbindungsleitung (34) von der Abzugsleitung (32) für das abgetrennte restozonhaltige Ozonträgergas des ersten Reaktions- und Entgasungsbehälters (20) in den unteren Bereich (29) eines nächsten Reaktions- und Entgasungsbehälters (30) führt.

19. Anlage nach einem der Ansprüche 11 bis 18, **dadurch gekennzeichnet**, daß die Einrichtung zur Einleitung des ozonhaltigen Gases in die zu behandelnde Flüssigkeit eine im Zulauf (1) der Flüssigkeit angeordnete Einleitungsvorrich tung (6) umfaßt.

20. Anlage nach einem der Ansprüche 11 bis 19, **dadurch gekennzeichnet**, daß die Einrichtung zur Einleitung des ozonhaltigen Gases in die zu behandelnde Flüssigkeit eine in einer von den Ableitungen (25,35) der Reaktions- und Entgasungsbehälter (20,30) in den Zulauf (1) zurückführende Rückführleitung (9) angeordnete Einleitungsvorrichtung (8) umfaßt.

## Claims

1. Process for treating aqueous liquids containing harmful materials which are difficult to decompose, by wet oxidation with ozone-containing gas and subsequent ozone-UV treatment,
characterised in that
the ozone containing gas is generated from oxygen-containing gas in an ozone generator by static discharge
the ozone-containing gas is introduced into the aqueous liquid under elevated pressure of a few bar in a part of the installation which is not exposed to the UV radiation.
the undissolved ozone-containing gas is separated out before the UV radiation,
the aqueous liquid is maintained under the elevated pressure in the introduction of the ozone-containing gas and on separation of the undissolved ozone-containing gas,
the substantially gas-bubble-free aqueous liquid containing ozone in absorbed form is irradiated with UV light for the purpose of simultaneous formation of radicals and oxidation with radicals in the passage through apparatus generating UV light,
and that the aqueous liquid is treated many times in a circuit, the flow of liquid conducted through the circuit being greater than the continuous incoming and outgoing flow of liquid.

2. Process according to Claim 1, characterised in that the introduction of the ozone-containing gas takes place with the aid of an injector into the feed-in of the liquid to be treated or to a part of the feed-in.

3. Process according to Claim 1 or 2, characterised in that the separated-out ozone-carrying gas containing residual ozone is allowed to react with the fluid again in order to achieve a residual ozone reaction.

4. Process according to one of Claims 1 to 3, characterised in that technical oxygen is used for ozone generation and the oxygen gas containing residual ozone is dried after separation from the liquid and is used again for generating ozone.

5. Process according to one of Claims 1 to 4, characterised in that the UV irradiation is performed with UV light of different wave lengths.

6. Process according to Claim 5, characterised in that the irradiation of a predetermined volume of liquid takes place simultaneously at different wave lengths.

7. Process according to Claim 5, characterised in that the irradiation of a predetermined volume of liquid takes place with different wavelengths in sequence.

8. Process according to one of Claims 1 to 7, characterised in that the pH value of the liquid to be treated is regulated in order to increase its reactivity.

9. Process according to one of Claims 1 to 8, characterised in that the liquid to be treated is heated in order to increase the speed of reaction.

10. Process according to one of Claims 1 to 9, characterised in that after the introduction of the ozone-containing gas and before the combined ozone-UV action, the liquid is subjected to a flocculation filtration or a flocculated sedimentation.

11. Installation for carrying out the process according to claims 1 to 10
with an inlet (1) for the aqueous liquid to be treated and an outlet (31) for the ready-treated aqueous liquid,
with a source of ozone-containing gas
with means for introducing the ozone-containing gas into the liquid to be treated
and with means, following the means for introducing the ozone-containing gas, for irradiating the liquid under treatment with UV light,
characterised in that
the source of ozone-containing gas is an ozone generator (10) in which the ozone-containing gas is generated from an oxygen-containing gas by static discharge,
that means (6, 8; 46) are provided for introducing the ozone-containing gas into the aqueous liquid under treatment under elevated pressure of a few bar,
that means (40, 50;80, 90) which follow the means (6, 8;46) are provided for irradiating the liquid under treatment with UV light,
that a reaction and de-gassing vessel (20;60, 70) is arranged between the means (6, 8;46)
and that for guiding the aqueous liquid through the means (6, 8;46) and (40, 50;80, 90) and the reaction and de-gassing vessel (20, 30;60, 70) there is provided a circuit in which a greater quantity of liquid can be conducted than enters at the inlet (1) and leaves at the outlet (31) in the continuous operation.

12. Installation according to Claim 11, characterised in that the reaction and de-gassing vessel arrangement comprises reaction and de-gassing vessels (20, 30, 60, 70) which are formed as double vessels with two vessels (13, 14) one inside the other, of which the outer vessel (13) is closed apart from a discharge pipe (32, 34, 62, 53) in its upper region (21, 36, 49, 51) for the separated-out ozone-carrying gas and the inner vessel (14) is open at the top and forms a weir (16), the feed pipe (12, 42, 44) for the liquid to be treated being conducted from above into the lower region (17) of the inner vessel (14) and the discharge pipes (24, 25, 35, 43, 45, 47) emerging from the lower region (23, 79) of the outer vessel (13).

13. Installation according to Claim 11 or 12, characterised in that discharge pipes (24, 31 and 25, 35, 47) both for the liquid destined for discharge and also for the liquid destined for recirculation, which is fed back into the inlet through a return pipe (26, 47), are provided in the lower region (23) of the outer vessel (13).

14. Installation according to one of Claims 11 to 13, characterised in that a first reaction and de-gassing vessel (20, 60) is followed by at least one further reaction and de-gassing vessel (30, 70), of which the feed pipe (28, 44) is connected to the outlet pipe (24, 43) of the first reaction and de-gassing vessel (20, 60).

15. Installation according to Claim 14, characterised in that the means for irradiating the liquid under treatment comprise an irradiation unit (40) inserted between the two reaction and de-gassing vessels (20, 30).

16. Installation according to one of Claims 11 to 15, characterised in that the means for irradiating the liquid under treatment comprise an irradiation unit (80) arranged in the outlet pipe (45) of the last reaction and de-gassing vessel (70).

17. Installation according to one of Claims 11 to 16, characterised in that the means (90) for irradiating the liquid under treatment comprise an irradiation unit (90) arranged in a feed back pipe (9, 59) leading back from the outlet pipes (25, 35;47) into the inlet.

18. Installation according to one of Claims 14 to 17, characterised in that a connecting pipe (34) from the discharge pipe (32) for separated-out ozone-carrying gas containing residual ozone from the first reaction and de-gassing vessel (20) leads into the lower region (29) of a subsequent reaction and de-gassing vessel (30).

19. Installation according to one of Claims 11 to 18, characterised in that the means for introducing ozone-containing gas into the liquid under treatment comprise an introduction device (6) arranged in the feed-in pipe (1) for liquid.

20. Installation according to one of Claims 11 to 19, characterised in that the means for introducing ozone-containing gas into the liquid under treatment comprise an introduction device (8) arranged in a return feed pipe (9) leading back from the discharge pipes (25, 35) of the reaction and de-gassing vessel (20, 30) into the feed-in pipe (1).

## Revendications

1. Procédé pour le traitement de liquides chargés de substances polluantes aqueuses, difficilement dégradables par oxydation humide avec du gaz contenant de l'ozone et traitement consécutif par UV et ozone,
caractérisé en ce que
• le gaz contenant de l'ozone est produit à partir d'un gaz contenant de l'oxygène dans un générateur à ozone par décharge silencieuse,
• le gaz contenant de l'ozone est introduit dans une partie de l'installation qui n'est pas soumise à l'irradiation par les UV, sous une pression augmentée de quelques bars, dans le liquide,
• le gaz contenant de l'ozone non dissout est séparé avant l'irradiation par les UV,
• le liquide est maintenu sous pression accrue au cours de l'introduction du gaz contenant de l'ozone et au cours de la séparation du gaz contenant de l'ozone non dissout,
• le liquide renfermant de l'ozone sous forme absorbée, essentiellement dépourvu de bulle de gaz est irradié par les UV dans le but de la formation de radicaux et de l'oxydation simultanément par des radicaux dans le passage à travers un appareillage produisant de la lumière UV, et
• le liquide est traité plusieurs fois dans un circuit dans lequel le courant de liquide mis en circuit est plus grand que le courant de liquide amené et évacué en continu.

2. Procédé selon la revendication 1,
caractérisé en ce que
l'introduction du gaz contenant de l'ozone s'effectue à l'aide d'un injecteur dans le courant d'amenée du liquide à traiter ou dans une partie du courant d'amenée.

3. Procédé selon la revendication 1 ou la revendication 2,
caractérisé en ce que
le gaz support d'ozone contenant l'ozone résiduel séparé est laissé réagir en vue de l'obtention d'une réaction de l'ozone résiduel à nouveau avec le liquide.

4. Procédé selon l'une des revendications 1 à 3,
caractérisé en ce qu'
on utilise de l'oxygène industriel en vue de l'obtention d'ozone et qu'on sèche l'oxygène gazeux contenant l'ozone résiduel après la séparation du liquide, et on l'utilise à nouveau en vue de la production d'ozone.

5. Procédé selon l'une des revendications 1 à 4,
caractérisé en ce que
l'irradiation par les UV se fait avec de la lumière UV de différentes longueurs d'onde.

6. Procédé selon la revendication 5,
caractérisé en ce que
l'irradiation d'un volume déterminé de liquide se fait simultanément avec les longueurs d'ondes différentes.

7. Procédé selon la revendication 5,
caractérisé en ce que
l'irradiation d'un volume déterminé de liquide se fait avec les différentes longueurs d'ondes les unes après les autres.

8. Procédé selon l'une des revendications 1 à 7,
caractérisé en ce que
la valeur de pH du liquide à traiter est régulée, pour augmenter la réactivité de celui-ci même.

9. Procédé selon l'une des revendications 1 à 8,
caractérisé en ce que
le liquide à traiter est chauffé, pour augmenter la vitesse de réaction.

10. Procédé selon l'une des revendications 1 à 9,
caractérisé en ce qu'
après l'introduction du gaz contenant de l'ozone et avant l'action combinée ozone-UV, le liquide est soumis à une filtration par floculation ou à une sédimentation par floculation.

11. Installation pour la réalisation du procédé selon les revendications 1 à 10, avec un courant d'amenée (1) pour le liquide qui doit être traité et un courant de sortie (31) pour le liquide traité finalement,
• avec une source de gaz contenant de l'ozone,
• avec un dispositif pour introduction du gaz contenant de l'ozone dans le liquide à traiter, et
• avec un dispositif connecté à la suite au dispositif pour introduction du gaz contenant de l'ozone, en vue de l'irradiation du liquide à traiter avec la lumière UV,
caractérisée en ce que
• la source de gaz contenant de l'ozone est un générateur d'ozone (10), dans lequel le gaz contenant de l'ozone peut être produit à partir d'un gaz contenant de l'oxygène par décharge,
• un dispositif (6, 8 ; 46) est prévu en vue de l'introduction du gaz contenant de l'ozone dans le liquide à traiter, sous pression augmentée de quelques bars,
• le dispositif connecté à la suite du dispositif (6, 8 ; 46) est prévu en vue de l'irradiation du liquide à traiter avec la lumière UV,
• on dispose entre les dispositifs (6, 8 ; 46) un réservoir de réaction et de dégazage (20, 60, 70) et,
• pour la conduite du liquide aqueux à travers les dispositifs (6, 8 ; 46) et (40, 50 ; 80, 90) et à travers les réservoirs de réaction et de dégazage (20, 30 ; 60, 70) un circuit est prévu, dans lequel une quantité de liquide plus grande peut être conduite que celle qui afflue en fonctionnement continu, au courant d'amenée (1) et est évacuée au courant de sortie (31).

12. Installation selon la revendication 11,
caractérisée en ce que
la disposition de réservoirs de réaction et de dégazage comprend des réservoirs de réaction et de dégazage (20, 30, 60, 70), qui sont réalisés sous forme de réservoirs doubles avec deux réservoirs placés l'un dans l'autre (13, 14), parmi lesquels le réservoir extérieur (13) est fermé jusqu'à une conduite d'évacuation (32, 34, 62, 53) débouchant dans la zone supérieure (21, 36, 49, 51) et destinée au gaz support d'ozone séparé, et le réservoir intérieur (14) est ouvert à son sommet et constitue un trop-plein (16), la conduite d'amenée (12, 42, 44) du liquide à traiter va du haut jusqu'à la zone inférieure (17) du réservoir intérieur (14) et les conduites de décharge (24, 25, 35, 43, 45, 47) partent de la zone inférieure (23, 79) du réservoir extérieur (13).

13. Installation selon les revendications 11 à 12,
caractérisée en ce qu'
il a été prévu dans la zone inférieure (23) du réservoir (13) extérieur, des conduites d'évacuation (24, 31 ou 25), 35, 47) aussi bien pour le liquide destiné à la décharge que pour le liquide destiné à la circulation, qui est ramené par une canalisation de retour (26, 47) dans l'entrée.

14. Installation selon l'une des revendication 11 à 13,
caractérisée en ce qu'
à la suite d'un premier réservoir de réaction et de dégazage (20, 60) on a connecté à la suite au moins un autre réservoir de réaction et de dégazage (30, 70), dont la conduite d'amenée (28, 44) est reliée à la conduite d'évacuation (24, 43) du premier réservoir de réaction et de dégazage (20, 60).

15. Installation selon la revendication 14,
caractérisée en ce que
l'appareillage d'irradiation du liquide à traiter comprend une unité d'irradiation (40) insérée entre les deux réservoirs de réaction et de dégazage (20, 30).

16. Installation selon l'une des revendications 11 à 15,
caractérisée en ce que
l'appareillage d'irradiation du liquide à traiter comprend une unité d'irradiation (80) dans la conduite d'évacuation (45) du dernier réservoir de réaction et de dégazage (70).

17. Installation selon l'une des revendications 11 à 16,
caractérisée en ce que
l'appareillage (90) d'irradiation du liquide à traiter comporte une unité d'irradiation (90) placé dans une conduite de retour (9, 59) retournant à une conduite d'évacuation (25, 35, 47) à la conduite d'amenée.

18. Installation selon l'une des revendications 14 à 17,
caractérisée en ce qu'
une conduite de liaison (34) conduit de la conduite d'évacuation (32) du gaz support d'ozone résiduel séparé, du premier réservoir de réaction et de dégazage (20) à la zone inférieure (29) d'un réservoir de réaction et de dégazage (30) voisin.

19. Installation selon l'une des revendications 11 à 18,
caractérisée en ce que
l'installation pour l'introduction du gaz contenant de l'ozone dans le liquide à traiter comprend un dispositif d'introduction (6) placé à l'amenée (1) du liquide.

20. Installation selon l'une des revendications 11 à 19,
caractérisée en ce que
l'installation pour l'introduction du gaz contenant de l'ozone dans le liquide à traiter, comprend un dispositif d'introduction (8), disposé dans une conduite de retour (9) revenant des conduites d'évacuation (25, 35) des réservoirs de réaction et de dégazage (20, 30) dans la conduite d'amenée (1).
